# EUROPEAN PATENT APPLICATION

(11) **EP 1 857 106 A1**
(43) Date of publication of application: **21.11.2007**
(21) Application number: 06010042.7
(22) Date of filing: 16.05.2006
(51) Int. Cl.: A61K 31/381, A61K 31/137, A61K 31/4704, A61P 11/00, A61P 11/06, A61P 11/08, A61P 43/00

(54) **Combinations of erdosteine and beta-2 agonists for treating COPD**

(71) Applicant: EDMOND PHARMA S.R.L., 20157 Milano (IT)
(72) Inventor: Arbasino, Mario, 20157 Milano (IT); Assereto, Roberto, 20157 Milano (IT)
(74) Representative: Minoja, Fabrizio

(57) **Abstract**

The invention relates to a method for improving the reversibility of bronchial obstruction in a subject affected by chronic obstructive pulmonary disease comprising the administration to said subject of erdosteine in combination with a beta agonist.

## Description

The invention concerns a method for improving the reversibility of bronchial obstruction in a subject affected by chronic obstructive pulmonary disease comprising the administration to said subject of erdosteine in combination with a beta agonist.

The invention also concerns the combined use of erdosteine for the preparation of a medicament for improving the reversibility of bronchial obstruction in a subject affected by chronic obstructive pulmonary disease.

### Background of the invention

Bronchodilating drugs are widely used in respiratory pathologies associated with airways obstruction, such as asthma or chronic obstructive pulmonary disease (COPD) (1).

The most widely used bronchodilators are the beta 2-agonists such as bambuterol, clenbuterol, salbutamol, formoterol, salmeterol and many others.

These drugs are the first-line treatment of both asthma and COPD, but their efficacy in COPD is limited by the fact that the airway obstruction caused by the disease is by definition (2) only partially reversed by beta agonists. The reversibility of bronchial obstruction is assessed by mean of spirometry. If a patient has a ratio between Forced Espiratory Volume in 1 s (FEV1) and Forced Vital Capacity (FVC) less than 70% of the predicted value (for age, weight and height) the diagnosis of COPD can be possible. The parameter used for testing the reversibility is FEV1 that, after inhalation of the short acting β2 receptors agonist salbutamol, must be <80% of the predicted value. In the case of asthma, and not COPD, the values will be always >80% (2). β2 receptors agonists are therefore much less effective, in their bronchodilatory activity, in COPD than in other diseases like asthma, but they are still the front-line treatment also in COPD.

Erdosteine is a drug originally developed as a mucolytic and now available in more than 30 countries for the therapy of pathologies associated with mucus disorders. Erdosteine is rapidly metabolized with the opening of its tbiolactone ring that forms a compound with a free thiol group.

The free thiol group is responsible for the activities of erdosteine; it breaks the S-S bonds of mucoproteins thus decreasing the viscosity of mucus and facilitating its elimination from the airways. This activity on the mucus fluidity can be beneficial in the treatment of COPD. This beneficial effect, common also to other mucolytic drugs such as N-acetylcysteine and carboxymethyl cysteine, was demonstrated in clinical trials. Nonetheless, according to Martindale (3): "The use of mucolytics or expectorants is controversial. Although meta-analysis has suggested a modest benefit in reducing exacerbations and some guidelines allow for the use of mucolytics, other guidelines do not consider there to be sufficient evidence to recommend them". This means that mucolytic drugs may be used in the therapy of COPD but that their role is mostly considered as supportive and symptomatic, linked only to their beneficial activity on mucus viscosity. Furthemore, erdosteine and all the drugs belonging to the same class have no direct or indirect bronchodilating activity.

There are no available data about a possible pharmacological or clinical interaction (both positive or negative) between erdosteine and β-2 agonists as far as bronchodilatation is concerned; no data, furthermore, are present in the literature also with drugs belonging to the same therapeutic class such as, for example, N-acetylcysteine and carbocysteine.

A drug that could cause a complete reversal, or at least a major reversal of the bronchial obstruction, could well be the treatment of choice in COPD.

### Description of the invention

Surprisingly, it has been found that erdosteine, when administered with beta agonists, causes a reversal of the bronchial obstruction much more evident than that obtained with the beta agonist alone.

This effect has been found in a trial utilizing the test of bronchial reversibility with salbutamol before and after 4 and 10 days of therapy with erdosteine at the dose of 900 mg/day, that is the maximum dosage presently approved in humans. The standard protocol has been used: spirometry was performed before and 15-20 minutes after the administration of 200 *µ*g of salbutamol with a metered inhaler.

For reducing the bias, the study was double-blind against placebo and the patients selected were newly diagnosed COPD subjects never treated with β-2 agonists. 7 patients were treated with erdosteine and 7 with placebo in a random order.

At baseline, all the patients had only a partial reversibility (mean 2.5%) of bronchial obstruction calculated with the FEV1 (the absolute values were 1.5 liters before salbutamol and 1.537 liters after salbutamol), thus fully meeting the COPD definition. When the test was repeated at Day 4 of therapy, the reversibility of bronchial obstruction in the placebo group was not different from the baseline values (mean increase of FEV1: 0.9%), while in the erdosteine group there was a significant and unexpected increase of post-salbutamol FEV1 values in all the patients (mean increase of FEV1: 7.1%). The data were fully confirmed, with a further improvement, at the final control at Day 10 for the patients treated with erdosteine (mean increase of FEV1: 10.3%).

These findings have a great clinical significance. They provide in fact for the first time conclusive evidence that an improvement of the reversibility of bronchial obstruction may be obtained.

Moreover, since (β-2 agonists are at the basis of the therapy of COPD, a potentiation by erdosteine of their activity on bronchial obstruction, that is only partial by definition, is a definite improvement in the chronic therapy of these patients. The more important reversibility of bronchial obstruction allow better physical conditions for the patients with important improvements in their daily activities and finally a better quality of life.

The increase of FEV1 values obtainable by erdosteine and beta agonists administration is higher than 7%.

According to these findings, the invention provides a new therapeutic method for treating pathologies associated with non reversible or partially reversible bronchial obstruction, like for example chronic obstructive pulmonary disease (COPD).

Erdosteine can be administered orally in the dosage form of tablets, film-coated tablets, capsules, sachets, oral suspension or any other suitable oral form, or nasally in solutions for inhalation.

Capsules containing 150, 300 or 450 mg of erdosteine are preferably administered b.i.d or t.i.d at doses of 300 mg to 1500 mg/day, in combination, for example, with beta 2 agonists such as salbutamol, formoterol, tulobuterol, procaterol or salmeterol administered by inhalation at doses of 100 *µ*g from one to four times per day (salbutamol), 4.5 or 6 or 12 or 24 *µ*g by inhalation 2 times daily (formoterol) and 50 or 100 *µ*g twice a day by inhalation (salmeterol).

The invention also provides products containing erdosteine and a beta agonist as a combined preparation for the simultaneous, separate or sequential use.

The invention is illustrated by the following examples:

Example 1: Patient #1, female, age 55 years, treated with erdosteine; before therapy, FEV1 pre-salbutamol 1.81, post salbutamol 1.81. After 4 days of therapy, FEV1 pre salbutamol 1.85, post salbutamol 2.05 (+10.8%). After 10 days, FEV 1 pre-salbutamol 1.93, post salbutamol 2.13 (+9.4%).

Example 2: Patient #4, male, age 69 years, treated with erdosteine; before therapy, FEV1 pre-sallbutamol 1.19, post salbutamol 1.26 (+5.9%). After 4 days of therapy, FEV1 pre salbutamol 1.74, post salbutamol1.96 (+12.6%). After 10 days, FEV1 pre-salbutamol 1.69, post salbutamol 1.92 (+13%).

Example 3: Patient #3, male, age 72 years, treated with placebo; before therapy FEV1 pre-salbutamol 1.98, post-salbutamol 2.04 (+3%). After 4 days of therapy, FEV1 pre salbutamol 1.82, post-salbutamol 1.89 (+3.8%). After 10 days, FEV1 pre-salbutamol1.84, post-salbutamol 1.82 (-1.10%).

Example 4: Patient #5, male, age 45 years, treated with placebo; before therapy, FEV1 pre-salbutamol 1.82, post salbutamol 1.88 (+3.3%). After 4 days of therapy, FEV1 pre salbutamol, 1.84, post salbutamol 1.92 (+4.3%).%). After 10 days of therapy, FEV1 pre salbutamol 1.82, post-salbutamol 1.85 (+1.6%).

### REFERENCES

1. Tashkin DP and Cooper CB. The role of long-acting bronchodilators in the management of stable COPD. Chest, Vol 125, 249-259, 2004.
2. Pauwels RA, Buist AS et al. Global strategy for the diagnosis,management and prevention of chronic obstructive pulmonary disease. Am J. Respir Crit Care Med, Vol 163, 1256-1276, 2001.
3. Martindale (Editor: Sweetman SC) The complete drug reference, 34th Edition, Pharmaceutical Press (London), Page 779, 2005.

## Claims

1. A method for improving the reversibility of bronchial obstruction in a subject affected by chronic obstructive pulmonary disease comprising the administration to said subject of erdosteine in combination with a beta agonist.

2. A method according to claim 1, wherein erdosteine is administered orally at dosages ranging from 300 to 1500 mg/day.

3. A method according to claim 2 wherein erdosteine is administered orally at dosages ranging from 300 to 900 mg/day.

4. A method according to any one of claims 1 to 3 wherein the beta agonist is selected from salbutamol, formoterol, procaterol, salmeterol, tulobuterol.

5. A method according to claim 4 wherein post-salbutamol FEV1 values are increased as a consequence of erdosteine administration.

6. A method according to claim 5 wherein the increase is higher than 7%.

7. The combined use of erdosteine for the preparation of a medicament for improving the reversibility of bronchial obstruction in a subject affected by chronic obstructive pulmonary disease.

8. The use according to claim 7 wherein erdosteine is administered orally.

9. The use according to claim 7 or 8 wherein the beta agonist is selected from salbutamol, formoterol, procaterol, salmeterol, tulobuterol.

10. Products containing erdosteine and a beta agonist as a combined preparation for the simultaneous, separate or sequential use.
